(19) Europäisches Patentamt
European Patent Office
Office européen des brevets

(11) Veröffentlichungsnummer: **0 181 409**
**B1**

# (12) EUROPÄISCHE PATENTSCHRIFT

(45) Veröffentlichungstag der Patentschrift: **08.02.89**

(51) Int. Cl.⁴: **C 07 C 177/00**, A 61 K 31/557

(21) Anmeldenummer: **85902719.5**

(22) Anmeldetag: **24.04.85**

(86) Internationale Anmeldenummer: **PCT/SU 85/00029**

(87) Internationale Veröffentlichungsnummer: **WO 85/04875 (07.11.85 Gazette 85/24)**

## (54) 15(R+S)-FLUOR-11,15-DIDESOXYPROSTAGLANDIN-E1-ABKÖMMLINGE.

(30) Priorität: **24.04.84 SU 3731856**

(43) Veröffentlichungstag der Anmeldung: **21.05.86 Patentblatt 86/21**

(45) Bekanntmachung des Hinweises auf die Patenterteilung: **08.02.89 Patentblatt 89/6**

(84) Benannte Vertragsstaaten: **CH DE GB IT LI**

(56) Entgegenhaltungen:
**WO-A-83/00844**
**WO-A-84/02483**
**DE-A-3 026 696**
**FR-A-2 193 612**
**GB-A-1 339 742**
**SE-B-315 742**
**SE-B-338 439**
**US-A-4 338 332**
**US-A-4 410 720**
**US-A-4 461 917**

(73) Patentinhaber: **INSTITUT ORGANICHESKOGO SINTEZA AKADEMII NAUK LATVIISKOI SSR, ul. Aizkraukles, 21, Riga 226006 (SU)**
Patentinhaber: **INSTITUT BIOORGANICHESKOI KHIMII IMENI M.M. SHEMYAKINA AKADEMII NAUK SSR, ul. Miklukho-Maklaya, 16/10, Moscow, 117871 (SU)**
Patentinhaber: **MOSKOVSKY MEDITSINSKY STOMATOLOGICHESKY INSTITUT IMENI N.A. SEMASHKO, ul. Delegatskaya 20/1, Moscow, 103473 (SU)**

(72) Erfinder: **BEZUGLOV, Vladimir Vilenovich, ul. Khoromny tupik, 2/6-81, Moscow, 107078 (SU)**
Erfinder: **BERGELSON, Lev Davydovich, Lavrushinsky per., 17-20, Moscow, 109017 (SU)**
Erfinder: **LAKIN, Kapiton Mikhailovich, Leninsky pr., 60/2-149, Moscow, 117333 (SU)**
Erfinder: **MAKAROV, Vladimir Alexandrovich, ul. Gorkogo, 12-45a, Moscow, 103009 (SU)**
Erfinder: **KOVALEV, Sergei Gennadievich, ul. Vucheticha, 10-32, Moscow, 125206 (SU)**
Erfinder: **FREIMANIS, Yanis Fritsevich, ul. Stirnu, 55-12, Riga, 226059 (SU)**

(74) Vertreter: **Finck, Dieter et al, Patentanwälte v. Füner, Ebbinghaus, Finck Mariahilfplatz 2 & 3, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 181 409 B1

ACTORUM AG

## Beschreibung

*Gebiet der Technik*

Die Erfindung bezieht sich auf das Gebiet der Chemie, und betrifft insbesondere Derivate des 15-(R+S)-Fluor-11,15-didesoxyprostaglandins $E_1$.

*Vorhergehender Stand der Technik*

Gegenwärtig sind verschiedene Stoffe bekannt, die die Aggregation vom Thrombozyten herabsetzen beispielsweise Dipiridemole (M.D. Mashkovsky, Lekarstvennyje sredstva, Moskau, Medizin, 1984, B.I. S. 420-421).

Das genannte Präparat weist jedoch keine hohe Antiaggregationsaktivität auf.

Bekannt sind in ihrer chemischen Struktur nahe Derivate der 15-Fluorprostansäuren beispielsweise 15-Fluor-15-desoxyprostaglandin $E_1$ und 15-Fluor--15-desoxyprostaglandin $J_2$, die die Aggregationsfähigkeit der Thrombozyte vermindern. (US-Urheberschein Nr. 959 785, Kl. C07C 177/00, A 61K 31/557, 1982).

Diese Verbindungen verlieren jedoch unter Bedingungen der Inkubation mit dem mit Thrombozyten angereicherten Blutplasma in einem wesentlichen Masse ihre Antiaggregationsaktivität bereits in 30 bis 45 Minuten, was ihre Verwendung in medizinischen und biologischen Experimenten erschwert.

Die Synthese dieser Verbindungen besteht ausserdem aus mehreren Stufen und verläuft mit einer niedrigen Endausbeute am Fertigungsprodukt. In diesem Zusammenhang haben die genannten Verbindungen keine praktische Anwendung gefunden.

*Offenbarung der Erfindung*

Die erfindungsgemässen Derivate des 15(R+S)-Fluor-11,15-didesoxyprostaglandins $E_1$ sind neu und in Veröffentlichungen nicht beschrieben worden.

Der Erfindung liegt die Aufgabe zugrunde, neue Verbindungen zu entwickeln, die eine hohe Antiaggregationsaktivität mit prolongierter Wirkung aufweisen und die nach einer vereinfachten Technologie hergestellt werden.

Diese Aufgabe wurde dadurch gelöst, dass neue Derivate des 15(R+S)-Fluor-11,15-didesoxyprostaglandins $E_1$ erfindungsgemäss folgender allgemeiner Formel sind:

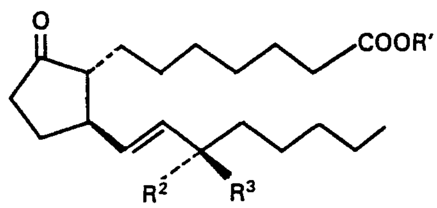

worin: R' = $CH_3$; $C_2H_5$; H; Na, $R^2$ oder $R^3$ = H oder Fluor sind ($R^2$ ist unterschiedlich zu $R^3$).

Die erfindungsgemässen Verbindungen stellen farblose zähflüssige Flüssigkeiten dar, die in der Regel in organischen Lösungsmitteln gut und im Wasser unwesentlich löslich sind. Eine Ausnahme bildet das Natriumsalz des 15(R+S)-Fluor-11,15-didesoxyprostaglandins $E_1$ (R' = Na), das im Wasser mässig löslich ist.

Die erfindungsgemässen Verbindungen weisen eine prolongierte Antiaggregationswirkung auf.

Die Antiaggregationswirkung der erfindungsgemässen Derivate des 15-(R+S)-Fluor-11,15-didesoxyprostaglandins $E_1$ wurde in Versuchen in vitro nach der Born-Methode untersucht.

Dem 0,9 ml mit Thrombozyten angereicherten Blutplasma setzte man 0,1 ml der Lösung des fluorierten Derivates des Prostaglandins hinzu, das durch die Auflösung einer Aliquote der Standardlösung des Prostaglandins im Dimethylsulfoxid (10 mg/ml) in 0,1 ml der physiologischen Lösung hergestellt wird und 5 µg des Stoffes enthält, in der 1. Probe Methylester des 15(R+S)-Fluor-11,15-didesoxyprostaglandins $E_1$, in der 3. Probe — Methylester des 15-Fluor-15-desoxyprostaglandins $E_1$, in der 4. Probe — Methylester des 15-Fluor-15-desoxyprostaglandins $J_2$. Die Proben inkubierte man 10 Minuten lang bei Raumtemperatur, dann setzte man Adenosindiphosphat in der Endkonzentration von 2 g/ml hinzu. In der Kontrolle setzte man anstelle einer Aliquote des Prostaglandins eine physiologische Lösung hinzu, die die gleiche Menge des Dimethylsulfoxids enthielt. In der 1. Probe wurde eine Senkung der Aggregation der Thrombozyte um 80%, in der zweiten Probe — um 85%, in der dritten Probe — um 85% und in der vierten Probe — um 79% gegenüber der Kontrolle nachgewiesen. In 60 Minuten in der dritten Probe und in 120 Minuten in der vierten Probe kehrte die Aggregation der Thrombozyte, die mit dem Adenosindiphosphat induziert wurde, wieder zum Ausgangsniveau zurück. Zu dem gleichen Zeitpunkt (60 Minuten) wurde in der ersten und in der zweiten Probe eine Senkung der Aggregation der Thrombozyte um 30% und mehr im Vergleich zur Kontrolle festgestellt. Diesen Effekt beobachtete man für das Methylester des 15-(R+S)-Fluor-11,15--didesoxyprostaglandins $E_1$ während 120 Minuten.

Unter den ähnlichen Bedingungen beträgt die maximale Senkung der Aggregation von Thrombozyten, die durch das bekannte Präparat, Dipiridomol, hervorgerufen wird, 48% im Vergleich zur Kontrolle. Die Ergebnisse der Prüfungen sind in den Tabellen 1 und 2 aufgeführt.

Somit weisen die erfindungsgemässen Derivate des 15(R+S)-Fluor-11,15-didesoxyprostaglandins $E_1$ eine höhere Antiaggregationsaktivität gegenüber dem Dipiridomol-Präparat auf und übertreffen die bekannten Derivate der 15-Fluorprostansäuren in der prolongierten Wirkung ungefähr auf das 2,5fache bei der ungefähr gleichen Stärke des Antiaggregationseffektes.

TABELLE 1

Einfluss der Fluorprostaglandine und des Dipiridomols auf die Antiaggregationsfähigkeit der Thrombozyte

| Nr. der Probe | Die zu prüfende Verbindung 5 µg/ml | Hemmung der Aggregation der Thrombozyte (%) nach der vorherigen Inkubation der zu prüfenden Verbindung im Plasma während 10 Minuten |
|---|---|---|
| 1 | 2 | 3 |
| 1. | Methylester des 15(R+S)-Fluor-11,15-didesoxyprostaglandins $E_1$ ($R^1$ = $CH_3$) | 80 |
| 2. | Äthylester des 15(R+S)-Fluor-11,15-didesoxyprostaglandins $E_1$ ($R^1$ = $C_2H_5$) | 85 |
| 3. | Methylester des 15-Fluor-15-desoxyprostaglandins $E_1$ | 85 |
| 4. | Methylester des 15-Fluor-15-desoxyprostaglandins $J_2$ | 79 |
| 5. | 15(R+S)-Fluor-11,15-didesoxyprostaglandins $E_1$ ($R^1$ = H) | 74 |
| 6. | Natriumsalz des 15(R+S)-Fluor-11,15-didesoxyprostaglandins $E_1$ (R' = Na) | 67 |
| 7. | Dipiridomol | 48 |

TABELLE 2

Dauer der Antiaggregationswirkung der Fluorprostaglandine

| Nr. der Probe | Prostaglandin 5 µg/ml | Änderung der Antiaggregationswirkung (in %) zu dem Ausgangsniveau bei der vorherigen Inkubation der Prostaglandine im Plasma während (Minuten) | | | |
|---|---|---|---|---|---|
| | | 10 | 30 | 60 | 120 |
| 1. | Methylester des 15(R+S)-Fluor-11,15-didesoxyprostaglandins $E_1$ (R' = $CH_3$) | 100 | 75 | 50 | 50 |
| 2. | Äthylester des 15(R+S)-Fluor-11,15-didesoxyprostaglandins $E_1$ (R' = $C_2H_5$) | 100 | 62 | 35 | 30 |
| 3. | Methylester des 15-Fluor-15-desoxyprostaglandins $E_1$ | 100 | 23 | 0 | 0 |
| 4. | Methylester des 15-Fluor-15-desoxyprostaglandins $J_2$ | 100 | 63 | 20 | 0 |

Die erfindungsgemässen Verbindungen werden aus dem bekannten Methyl- oder Äthylester des 11-Desoxyprostaglandins $E_1$ durch die Behandlung der Lösungen dieser Ester in einem organischen Lösungsmittel mit Morpholinosulfotrifluorid bei einer Temperatur unter 0°C gewonnen. Die entstehenden Fluoride werden mittels Extraktion mit einem geeigneten organischen Lösungsmittel mit anschliessender Reinigung mittels Chromatografie am Silikagel herausgelöst. Im weiteren wird notwendigenfalls eine alkalische Verseifung der Ester durchgeführt.

Zum besseren Verständis der vorliegenden Erfindung werden nachstehende Beispiele für die Herstellung der erfindungsgemässen Verbindung angeführt.

*Beispiel 1*

100 mg (0,27 mMol) des Äthylesters des 11-Desoxyprostaglandins $E_1$ löst man in 10 ml des trockenen Methylenchlorids auf und setzt man die gewonnene Lösung bei Abkühlung auf −78°C und unter Vermischen im Argonmedium einer Lösung aus 140 µl des Morpholinosulfotrifluorids in 5 ml des Methylenchlorids hinzu. Das Gemisch rührt man 1 Stunde lang bei einer Temperatur von −78°C, setzt man 2,5 ml einer gesättigten Lösung des Ammoniumchlorids hinzu und lässt man unter dem ständigen Rühren bis auf die Raumtemperatur erwärmen. Dann verdünnt man die Reaktionslösung mit Wasser (7,5 ml), die wässrige Schicht trennt man ab und löst man mit Methylenchlorid heraus. Die zusammengeführten organischen Phasen wäscht man mit einer gesättigten Lösung des Natriumbikarbonats (25 ml), mit Wasser bis zum neutralen pH-Wert, dann mit einer gesättigten Natriumchloridlösung (15 ml) und trocknet man mit Natriumsulfat. Das Trocknungsmittel wird abgefiltert, das Filtrat eingedampft, der Rückstand mit Hexan aufgelöst und an einer Säule mit 10 g Silikagel (100 bis 160 µm) im Gradientsystem Hexan-Äthylazetat chromatografiert.

Man erhält 80,7 mg (81%, bezogen auf den theoretischen Wert) Äthylester des 15(R+S)-Fluor--11,15-didesoxyprostaglandins $E_1$, das ein farbloses zähflüssiges Öl darstellt, $R_f$ 0,65 (Silufol, ein Fleck, Eluant Benzol-Äthylazetat 7:1), Massenspektrum (m/z): 368 (M, 2), 348 (M-HF, 7), 323 ($M-C_2H_5O$, 3), 322 ($M-C_2H_5O$, 7), 303 (15), 302 (M-HF $-C_2H_5OH$, 15), 212 (15), 192 (30), 109 (100%), F-NMR-Spektrum (δ, Deuterochloroform): — 170,25 (m., Intensität 2,5), — 171,47 (m., Intensität 1), das Verhältnis von 15(S)- und 15(R)-Epimeren beträgt 2,5.

*Beispiel 2*

Eine Lösung aus 20 mg (0,059 mMol) des 11-Desoxyprostaglandins $E_1$ in 2 ml des Diäthylesters behandelt man unter Abkühlung auf 0°C mit dem Überschuss einer Diazomethan-Esterlösung. In 30 Minuten wird das Reaktionsgemisch bis zur Trocknung eingedampft, der Rückstand in 2 ml Methylenchlorid aufgelöst und, wie im Beispiel 1 beschrieben, unter Verwendung von 30 µm des Morpholinosulfotrifluorids fluoriert. Nach der entsprechenden Behandlung des Reaktionsgemisches löst man das Rohprodukt in Hexan auf und chromatografiert an einer Säule mit 3 g Silikagel (100 bis 160 µm) im Gradientsystem Hexan-Ester. Man erhält 14,5 g (70%, bezogen auf den theoretischen Wert) des Methylesters des 15-(R+S)-Fluor-11,15-didesoxyprostaglandins $E_1$, das ein farbloses zähflüssiges Öl darstellt, $R_f$ 0,37 (Silufol, ein Fleck, Hexan-Ester, 2:1), Massenspektrum (m/z): 354 (M, 5), 335 (M-F, 5), 334 (M-HF, 13), 323 ($M-CH_3O$, 3), 322 ($M-CH_3OH$, 7), 303 (18), 302 (M-HF $-CH_3OH$, 20), 212 (40), 192 (47), 143 (26), 109 (100%), $^1$H-NMR-Spektrum (δ, Deuterochloroform): 5,65 (2H, m., $C_{(13)}H$, $C_{(14)}H$; 4,85 (IH, d.m., $J_{HF}$ 48 Hz, $C_{(15)}H$); 3,61 (3H I., $C_{(1)}OCH_3$); 2,4 (2H, m., $C_{(10)}H$); 2,25 (2H. t., $C_{(8)}H$, $C_{(12)}H$; 2,1 (2H, d.d., $C_{(2)}H_{(2)}$; 0,89 (3H, t., $C_{(19)}CH_3$), $^{19}$F-NMR-Spektrum (δ, Deuterochloroform): — 170,3 (m., Intensität 2,5), — 171,5 (m., Intensität 1), das Verhältnis von 15(S)- und 15(R)-Epimeren beträgt 2,5.

*Beispiel 3*

Man behandelt eine Lösung aus 55 mg (0,16 Mol) des Äthylesters des 15(R+S)-Fluor-11,15-didesoxyprostaglandins $E_1$ in 3 ml eines Tetrahydrofuran-Methanol-Gemisches (1:2) mit 2 ml 1N wässrigen Natriumhydroxids und lässt man das gewonnene Gemisch bei Raumtemperatur 18 Stunden lang stehen. Die Reaktionslösung säuert man mit 1N Salzsäure bis auf einen pH-Wert von 2 bis 3 an, entfernt man mittels Eindampfung organische Lösungsmittel und den Rückstand löst man mit Äthylazetat (3 × 15 ml) heraus. Die zusammengeführten organischen Extrakte wäscht man mit Wasser bis zur neutralen Reaktion, mit einer gesättigten Lösung des Natriumchlorids (10 ml) und trocknet man mit Natriumsulfat. Das Trocknungsmittel filtriert man ab, das Filtrat wird eingedampft. Den Rückstand reinigt man mittels Chromatografie an 6 g Silikagel (100 bis 160 µm) im Gradientsystem, Chloroform-Äthylazetat. Man erhält 47 mg (92%, bezogen auf den theoretischen Wert) des 15(R+S)-Fluor-11,15-didesoxyprostaglandins $E_1$, das ein farbloses dickflüssiges Öl darstellt, $R_f$ 0,75 (Silufol, ein Fleck, Benzol-Dioxan-Essigsäure, 40:10:1). Massenspektrum des Methylesters, das durch die Behandlung 1 mg des 15-(R+S)-Fluor-11,15-didesoxyprostaglandins $E_1$ mit Diazomethan-Esterlösung gewonnen wird, ist dem im Beispiel 2 angeführten Massenspektrum identisch.

*Beispiel 4*

Einer Lösung aus 5 mg (0,014 mMol) des 15-(R+S)-Fluor-11,15-didesoxyprostaglandins $E_1$ in 5 ml Äthanol setzt man 0,375 ml einer wässrigen Natriumkarbonat-Lösung (5 mg/ml) hinzu. Das Gemisch wird 1 Stunde lang bei Raumtemperatur stehengelassen und bis zur Trocknung eingedampft. Man erhält 5,2 mg des Natriumsalzes des 15(R+S)--Fluor-11,15-didesoxyprostaglandins $E_1$.

*Industrielle Anwendbarkeit*

15-Fluor-didesoxyprostaglandine $E_1$ sind chemisch stabile Analogone des natürlichen Prostaglandins $E_1$, eines der aktivsten Antiaggregationsfaktoren im Organismus der Säugetiere. Die genannten Verbindungen sind ausserdem gegenüber der Prostaglandindehydrogenase, einem Ferment, das den Zerfall der Prostaglandine im Organismus iniziiert, stabil. Festgestellt wurde ebenfalls, dass 15-Fluor--didesoxyprostaglandine $E_1$ eine mittelmässige Fähigkeit besitzen, die Blutgefässe, hauptsächlich Koronargefässe, bei den Versuchstieren zu erweitern. Aufgrund der genannten Eigenschaften und in Verbindung mit geringer Toxizität von > 25 mg/kg können die erfindungsgemässen Verbindungen als Inhibitoren der Aggregation von Thrombozyten bei der Konservierung von Blut oder von Thrombozyt-Plasma und vielleicht auch bei Operationen unter Einsatz der künstlichen Blutzirkulation Anwendung finden. Der positive Effekt der 15-Fluor-didesoxyprostaglandine $E_1$ auf die Gefässe in Verbindung mit der Antiaggregationswirkung kann sich bei der Behandlung einiger Herz-Kreislauferkrankungen als nützlich erweisen.

**Patentanspruch**

Derivate des 15(R+S)-Fluor-11,15-didesoxyprostaglandins $E_1$ allgemeiner Formel:

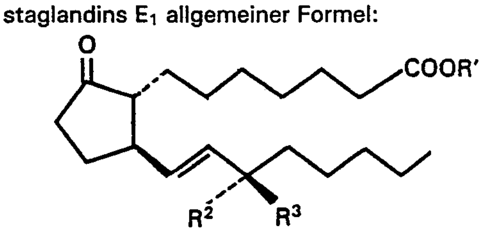

worin R' = Äthyl, Methyl, Wasserstoff, Natrium ist; $R^2$ oder $R^3$ = Wasserstoff oder Fluor sind ($R^2$ ist unterschiedlich zu $R^3$).

**Claim**

Derivatives of the 15(R+S)-fluorine-11,15-didesoxyprostaglandins $E_1$ of general formula:

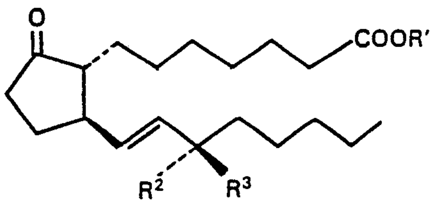

in which R' is ethyl, methyl, hydrogen, sodium; $R^2$ or $R^3$ are hydrogen or fluorine ($R^2$ differs from $R^3$).

**Revendication**

Dérivés de la 15(R+S)-fluoro-11,15-didésoxyprostaglandine $E_1$ de formule générale:

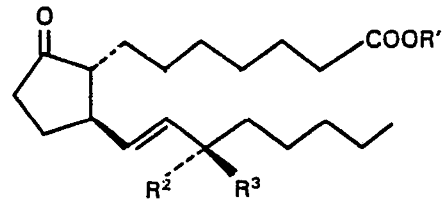

dans laquelle R' est éthyle, méthyle, hydrogène, sodium; $R^2$ ou $R^3$ sont hydrogène ou fluor ($R^2$ est différent de $R^3$).